# EUROPEAN PATENT SPECIFICATION

(11) **EP 4 517 337 B1**
(45) Date of publication and mention of the grant of the patent: **15.04.2026**
(21) Application number: 23724898.4
(22) Date of filing: 28.04.2023
(51) Int. Cl.: G01Q 30/04, G01Q 60/42, G01Q 60/32, G06V 20/69, G06N 3/045, G06V 10/82, G06N 3/0442, G06N 3/0464, G06N 3/082, G06N 3/09, G16C 20/20, G16C 20/70

(54) **A COMPUTER-IMPLEMENTED METHOD FOR IDENTIFYING A MOLECULE FROM ATOMIC FORCE MICROSCOPY IMAGES AND GENERATING THE NAME OF SAID MOLECULE ACCORDING TO THE IUPAC NOMENCLATURE**
COMPUTERIMPLEMENTIERTES VERFAHREN ZUR IDENTIFIZIERUNG EINES MOLEKÜLS AUS BILDERN DER RASTERKRAFTMIKROSKOPIE UND ZUR ERZEUGUNG DES NAMENS DIESES MOLEKÜLS GEMÄSS DER IUPAC-NOMENKLATUR
PROCÉDÉ MIS EN OEUVRE PAR ORDINATEUR POUR IDENTIFIER UNE MOLECULE À PARTIR D'IMAGES DE MICROSCOPIE À FORCE ATOMIQUE ET GENERATION DU NOM DE LADITE MOLECULE SELON LA NOMENCLATURE IUPAC

(30) Priority: 29.04.2022 ES 202230398
(43) Date of publication of application: 05.03.2025
(73) Proprietor: Universidad Autónoma de Madrid, 28049 Madrid (ES)
(72) Inventor: PÉREZ PÉREZ, Rubén, 28049 Madrid (ES); CARRACEDO COSME, Jaime, 28049 Madrid (ES)
(74) Representative: Pons IP
(86) International application number: PCT/ES2023/070276
(87) International publication number: WO 2023/209264

(56) References cited:
- BENJAMIN ALLDRITT ET AL: "Automated Structure Discovery in Atomic Force Microscopy", ARXIV.ORG, CORNELL UNIVERSITY LIBRARY, 201 OLIN LIBRARY CORNELL UNIVERSITY ITHACA, NY 14853, 24 May 2019 (2019-05-24), XP081546906
- RAJAN KOHULAN ET AL: "STOUT: SMILES to IUPAC names using neural machine translation", JOURNAL OF CHEMINFORMATICS, vol. 13, no. 1, 27 April 2021 (2021-04-27), XP093052797, Retrieved from the Internet <URL:https://link.springer.com/article/10.1186/s13321-021-00512-4/fulltext.html> DOI: 10.1186/s13321-021-00512-4

## Description

The invention relates to a computer implemented method for identifying a molecule from Atomic Force Microscopy images by generating the name of said molecule according to the IUPAC nomenclature using two trained Multimodal Recurrent Neural Networks.

The present invention is therefore of interest in the areas of nanotechnology, particularly in areas related to on-surface chemical reactions and therefore of interest for the Atomic Force Microscopy users and manufacturers.

### STATE OF ART

Scanning Probe Microscopes have played a key role in the development of nanoscience as the fundamental tools for the local characterization and manipulation of matter with high spatial resolution. In particular, Atomic Force Microscopy (AFM) operated in its frequency modulation (FM) mode allows the characterization and manipulation of all kind of materials at the atomic scale. This is achieved measuring the change in the frequency of an oscillating tip due to its interaction with the sample. When the tip apex is functionalised with inert closed-shell atoms or molecules, particularly with a CO molecule, the resolution is dramatically enhanced, providing access to the inner structure of molecules.

This outstanding contrast arises from the Pauli repulsion between the CO probe and the sample molecule modified by the electrostatic (ES) interaction between the potential created by the sample and the charge distribution associated with the oxygen lone pair at the probe. In addition, the flexibility of the molecular probe enhances the saddle lines of the total potential energy surface (PES) sensed by the CO. These high-resolution AFM (HR-AFM) capabilities have made possible to visualize frontier orbitals, to determine bond order potentials and charge distributions, and have opened the door to track and control on-surface chemical reactions.

Despite these impressive achievements one of the most important goals remains elusive: the molecular recognition. That is, the ability of naming a certain molecule exclusively by means of HR-AFM observations.

Molecules have been identified combining AFM with other experimental techniques like scanning tunnelling microscopy (STM) or Kelvin probe force microscopy (KPFM) and with the support of theoretical simulations ("Noncontact atomic force microscopy: Bond imaging and beyond" Q. Zhong, X. Li, H. Zhang, L. Chi, Surf. Sci. Rep. 75, 100509 (2020)).

Chemical identification by AFM of individual atoms at semiconductor surface alloys was achieved using reactive semiconductor apexes. In that case, the maximum attractive force between the tip apex and the probed atom on the sample carries information of the chemical species involved in the covalent interaction. However, the scenario is rather different when using tips functionalised with inert CO molecules where the main AFM contrast source is the Pauli repulsion, and the images are strongly affected by the probe relaxation. So far, the few attempts to discriminate atoms in molecules by HR-AFM have been based either on differences found in the tip-sample interaction decay at the molecular sites or on characteristic image features associated with the chemical properties of certain molecular components. For instance, sharper vertices are displayed for substitutional N atoms on hydrocarbon aromatic rings due to their lone pair. Furthermore, the decay of the CO-sample interaction over those substitutional N atoms is faster than over their neighbouring C atoms. Halogen atoms can also be distinguished in AFM images thanks to their oval shape (associated to their σ-hole) and to the significantly stronger repulsion compared to atoms like nitrogen or carbon. However, even these atomic features depend significantly on the molecular structure and cannot be only associated to a certain species but to its moiety in the molecule. The huge variety of possible chemical environments renders the molecular identification by a mere visual inspection by human eyes an impossible task.

Artificial Intelligence (AI) techniques are precisely optimized to deal with this kind of subtle correlations and massive data. Deep learning, with its outstanding ability to search for patterns, is nowadays routinely used to classify, interpret, describe and analyse images, providing machines with capabilities hitherto unique to human beings or even surpassing them in some tasks. In our previous work ("A Deep Learning Approach for Molecular Classification Based on AFM Images" J. Carracedo-Cosme, C. Romero-Muñiz, R. Pérez, Nanomaterials 11, 1658 (2021)), we restricted ourselves to essentially flat molecules and tested the potential of deep learning techniques to classify 60 different organic molecules from their constant-height AFM images. Although encouraging, the clear success of this proof of concept does not provide a solution to the general problem of molecular identification. The classification approach can only identify molecules included in the training data set. Given the rich complexity provided by organic chemistry, even an extremely large data set, that already poses fantastic computational challenges (as the output vector has the dimension of the number of molecules in the dataset), would fail to classify many of the already known or possibly synthesized molecules of interest. Alldritt et al., "Automated Structure Discovery in Atomic Force Microscopy",ARXIV.ORG, CORNELL UNIVERSITY LIBRARY, 201 OLIN LIBRARY CORNELL UNIVERSITY ITHACA, NY 14853, 24 May 2019, XP081546906, discloses a computer implemented method for identifying an organic molecule from Atomic Force Microscopy images.

Kohlan et al., "STOUT: SMILES to IUPAC names using neural machine translation", JOURNAL OF CHEMINFORMATICS, vol. 13, no. 1, 27 April 2021, XP093052797, DOI: 10.1186/ s13321-021-00512-4 discloses a machine learning method for translating strings representing molecules to IUPAC names.

Therefore, it is needed to develop new methods for achieving a complete molecular identification (structure and composition) through atomic force microscopy imaging, including non-planar structures.

### DESCRIPTION OF THE INVENTION

The present invention refers to a computer implemented method for identifying a molecule from atomic force microscopy images by generating the name of said molecule according to the IUPAC nomenclature using a combination of two different trained Multimodal Recurrent Neural Networks (M-RNN_{A} and AM-RNN); each Multimodal Recurrent Neural Network (M-RNN_{A} and AM-RNN) comprising a convolutional neural network CNN component, a recurrent neural network RNN component and a multimodal component φ. Therefore, the object of the present invention is to provide a text (the name of the molecule according to IUPAC nomenclature) describing an image (acquired plurality of FM-AFM images).

The IUPAC nomenclature, the most widely accepted and used in science, is adopted as molecular descriptor in the present invention. The IUPAC name determines unambiguously the molecular composition and structure. This is done by defining a hierarchical keyword list to name functional groups that are written following a systematic syntax that defines the structural position of each moiety or group in the molecule. In order to convert the IUPAC nomenclature into a suitable computational language, the present invention defines a set of terms into which each IUPAC name is broken down. In the present invention the term "set of terms into which each IUPAC name is broken down" refers herein to a set of letters or symbols denoting molecular moieties, ligands or specifying positions of atoms used by the IUPAC nomenclature. Combinations of these terms in a specific order generates the names of the molecules according to the IUPAC nomenclature.

A combination of two different trained Multimodal Recurrent Neural Networks (M-RNN_{A} and AM-RNN) is used in the method of the present invention to generate the name of the molecule according to IUPAC nomenclature. First trained M-RNN_{A} determines the main chemical groups (main molecular moieties) that compose the molecule defining a keyword for each moiety (herein IUPAC attributes), whereas second trained AM-RNN predicts the remaining IUPAC terms and assembles said remaining IUPAC terms with IUPAC attributes determined by the trained M-RNN_{A} in the precise order giving rise to the IUPAC nomenclature of the structure of the molecule.

In the present invention, a "term" is a set of letters or symbols denoting molecular moieties, ligands or specific positions of atoms used by IUPAC nomenclature. Combinations of these terms produce the IUPAC names of molecules.

In the present invention, the term "IUPAC attributes" refers to a 100-element subset of the IUPAC terms that mostly describe atomic groups. Table 1 shows the terms for IUPAC decomposition. The elements above the double line are the subset of 100 terms considered as attributes. The grey cell does not correspond to any term, it has been colored in order to distinguish it from the term that spells an empty space between two words.

**Table 1**

| | | | | | | | | | |
|---|---|---|---|---|---|---|---|---|---|
| acen | acet | acid | acr | alde | alen | amate | amide | amido | amim |
| amine | amino | amo | ane | ani | anil | ano | anone | anthr | ate |
| ato | aza | aze | azi | azido | azin | azo | azol | benz | brom |
| but | carb | chlor | chr | cyclo | ene | eno | eth | fluor | form |
| furan | furo | hyde | hydr | ic | ida | ide | idin | idine | ido |
| imid | imidin | imine | imino | ind | ine | ino | iod | iso | itrile |
| ium | meth | mine | naphth | nitr | nium | nyl | oate | oic | ol |
| ole | oli | olin | oline | olo | om | one | oso | oxo | oxol |
| oxy | oxyl | oyl | phen | phth | prop | pter | purin | pyr | pyrrol |
| quin | sulf | thi | tri | urea | yl | ylid | yridin | zin | zine |
| dodeca | undeca | lambda | phosph | cinnam | xanth | porph | coron | deca | guan |
| hept | amic | pent | enal | octa | anal | nona | mido | nida | azet |
| tetr | ulen | anol | hypo | pine | ysen | anth | tere | acyl | yrin |
| inin | tris | pino | mid | hex | nia | bis | per | nio | pin |
| ite | rin | alo | en | di | 11 | yn | an | cy | de |
| 15 | 10 | 13 | 14 | 12 | in | 18 | 21 | az | al |
| bi | et | ep | id | ox | il | or | 16 | 17 | on |
| ( | 6 | ) | - | [ | a | ] | N | ' | 1 |
| 7 | 4 | 5 | 3 | 9 | 2 | 8 | H | | , |
| o | b | e | c | C | d | O | g | f | |

Quasar Science Resources S.L. - Universidad Autónoma de Madrid - Atomic Force Microscopy (QUAM-AFM) (https://doi.orq/10.21950/UTGMZ7) is used for training the first trained Multimodal Recurrent Neural Networks M-RNN_{A} network and the second trained Attribute Multimodal Recurrent Neural Network AM-RNN.

QUAM-AFM is a publicly available dataset of 165 million AFM images theoretically generated from 686,000 isolated molecules using 240 different combinations of AFM operational parameters (10 tip-sample distances, 6 different oscillation amplitudes, and 4 different values for the torsional stiffness of the CO molecule, that are known to depend on the details of the attachment of the molecule to the metal tip apex. QUAM-AFM also provides the ball-and-stick depictions of each molecule generated from the atomic coordinates. These depictions share the same scale used in the AFM images: if we superimpose the two images, each ball of the representation is centered on the position occupied by the atom it represents in the AFM images.

QUAM-AFM dataset includes organic molecules, discarding all other compounds that may not have purely molecular forms, like organic salts or inorganic compounds and polymers. The selected molecules contain the four basic elements of organic chemistry (carbon, hydrogen, nitrogen, and oxygen) plus some other less common elements which are still frequent on organic compounds like sulphur, phosphorus, and the halogen atoms (fluorine, chlorine, bromine, and iodine). The largest molecule in QUAM-AFM database has a total of eighty--five atoms.

Very small molecules, namely, those containing less than eight atoms have been discarded, as due to their extremely high surface mobility and huge variety of adsorption configurations, are not good candidates to be identified solely by means of AFM and therefore are not included in QUAM-AFM database. In addition, very large molecules having a structure that does not fit into a square--based cell with a side length of 24 Å Are also not forming part of the QUAM-AFM dataset.

The database QUAM-AFM is restricted to quasi--planar molecules, which display only height variations up to 1.83 Å along the z-axis in order to include aliphatic chains with *sp³* carbon atoms (methyl groups) as side groups. QUAM-AFM comprises a set of molecules that includes aliphatic, cyclic and aromatic compounds, in particular a large number of hydrocarbons (alkanes, alkenes, alkynes, etc.) together with all the traditional organic families (alcohols, thiols, ethers, aldehydes and ketones, carboxylic acids, amines, amides, imines, esters, nitriles, nitro and azo compounds, halocarbons and acyl halides, etc.).

The IUPAC names in QUAM-AFM images can be decomposed into a total of 199 terms. The maximum length of terms in the decomposition of the IUPAC names in QUAM-AFM is 57.

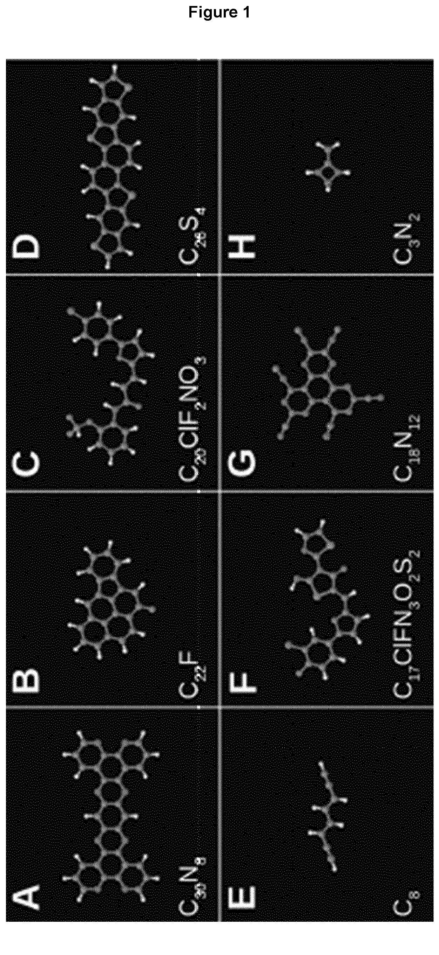

Please note, that a class of a molecule is defined herein by the type of atomic species that it contains and the number of repeated atoms of each of these species. A representative number of QUAM-AFM of each class is obtained excluding the hydrogen from the species list, so that molecules with completely different structures such as pyrazine, pyridazine, but-2-enedinitrile or butanedinitrile belong to the same class (C₄N₂). This results in a total of 2339 classes for the molecule structures considered in QUAM-AFM. See **Figure 1****.**

Multimodal Recurrent Neural Networks generate novel sentence descriptions to explain the content of images. A first trained Multimodal Recurrent Neural Networks M-RNN_{A} is used in the method of the present invention to obtain the main chemical groups, this is the main molecular moieties, that compose the molecule defining a keyword for each moiety herein IUPAC attributes.

A method for training the first trained Multimodal Recurrent Neural Network M-RNN_{A} comprising the following steps:
i) providing a first trained Multimodal Recurrent Neural Network M-RNN_{A}, said M-RNN_{A} comprising
   - a first convolutional neural network CNN/M-RNN_{A} component comprising a block of 3D convolutional layers and one or more dropout layers,
   - a first recurrent neural network RNN/M-RNN_{A} component comprising one or more embedding layers, one or more dropout layers, and one or more recurrent layers, wherein at least one recurrent layer is a Gated Recurrent Unit (GRU),
   - a first multimodal component φ/M-RNN_{A} comprising connecting layers with one or more dropout layers,
   - and an Image Data Generator
ii) pretraining the first convolutional neural network CNN/M-RNN_{A} component feeding said first recurrent neural network CNN/M-RNN_{A} component with a set of AFM images of known or predetermined molecules corresponding to the class of molecules that shares the same chemical composition (number of different chemical species and number of atoms for each specie, excluding the H atoms), for instance with the AFM images of known or predetermined molecules corresponding to a class of molecules that shares the same chemical composition in the database QUAM-AFM, wherein the shape and the contrast of said images and their variation with the height show the 3D positions and the size (chemical nature) of the atoms and the distance between said atoms in the organic molecule;
iii) feeding the first trained Multimodal Recurrent Neural Network M-RNN_{A} which first convolutional neural network CNN/M-RNN_{A} component is pretrained in step (ii) with a plurality of constant-height AFM greyscale images of known or predetermined organic molecules, for instance with the AFM images of the database QUAM-AFM, and, alternatively,
   - training the RNN/M-RNN_{A} component, updating its weights, while fixing the weights of the CNN/M-RNN_{A} component,
   - or training the CNN/M-RNN_{A} component, updating its weights, while fixing the weights of the RNN/M-RNN_{A} component,
thereby predicting the IUPAC attributes in an organic molecule.

The CNN/M-RNN_{A} component comprises a block of 3D convolutional layers and dropout layers and consists of a modification of the Inception ResNet V2 model (C. Szegedy, S. loffe, V. Vanhoucke, A. A. Alemi, 31rd Proc. AAAI Conf. on Artificial Intelligence (AAAI Press, Palo Alto, CA, USA, 2017), p. 4278-4284) where the 2D-convolutional layers is replaced by a block that includes two 3D convolutional layers -each one with 32 filters, (3,3,3) kernel size and (2,1,1) strides- in order to process the stack of 10 AFM images with various tip--sample distances, followed by a dropout layer. This dropout layer is essential to generalize to different images, such as the experimental ones. In addition, we have removed the last fully connected layer of the model, which is specific for the original classification task, obtaining an output vector v.

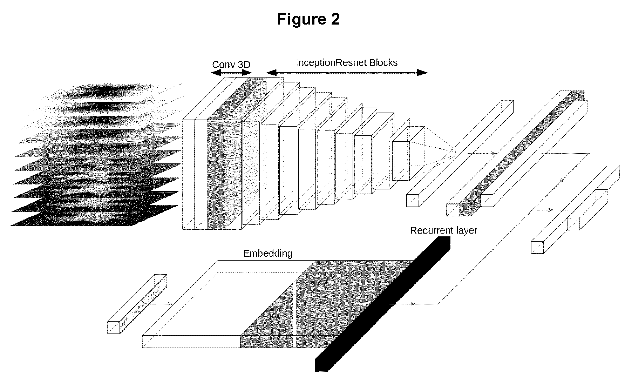

The RNN/M-RNN_{A} comprises an embedding layer, followed by a dropout layer, and ending with a recurrent layer (see **Figure 2****).** The attributes are encoded by assigning integer numbers from 1 to 100 to each attribute. The input of RNN/M-RNN_{A} is a vector of fixed size 19, the maximum number of different attributes in the names of the molecules in QUAM-AFM (18) plus the *startseq* token. In the first step, it will only contain one integer number designating the *startseq* token, while in the following steps it would include *startseq* plus the integer numbers associated with attributes predicted in previous steps. At each time step, the input is completed with zeros until we obtain a length of 19.

The embedding layer processes the input to represent each attribute in a vector space, transforming the input vector into a dense vector with real values that reflect the syntactic and semantic meaning of the attribute, placing similar attributes close together in the vector space, following the neural connections established during training. For example, the attributes represented closest to *brom* are *chlor, fluor* and *iod.*

The recurrent layer stores in its internal state information about the previous predictions. In attribute prediction, we use Gated Recurrent Unit (GRU) as the recurrent layer. GRU is computationally efficient and suitable for the short attribute chains to be predicted. It is necessary to introduce a dropout layer in between the embedding and recurrent layers in order to avoid overfitting during the training.

The multimodal component φ/M-RNN_{A} first processes the CNN output v in two fully connected layers with a dropout between them. The resulting vector is concatenated with the output vector of the RNN to feed two fully connected layers that produce a vector of probabilities. This final vector has 103 components: one hundred components are related to the attributes, one with the padding and two with the *startseq* and *endseq* tokens. The position of the larger component in the vector provides us with the prediction of a new attribute.

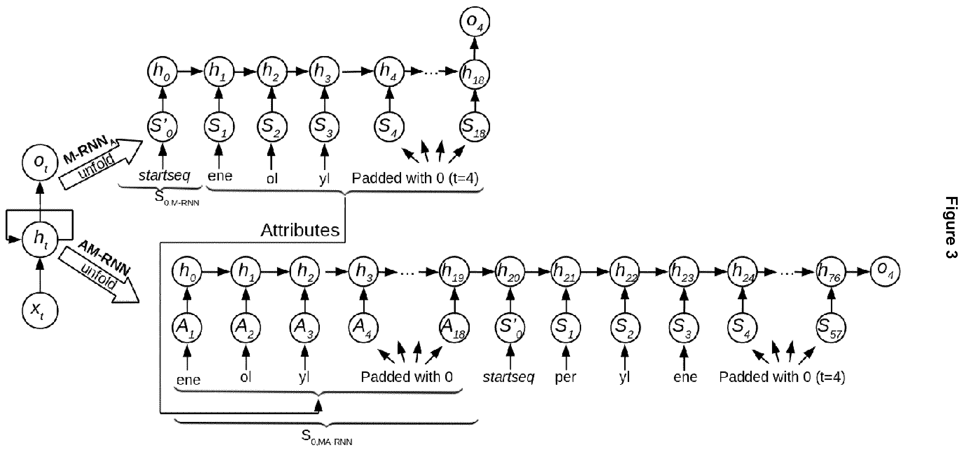

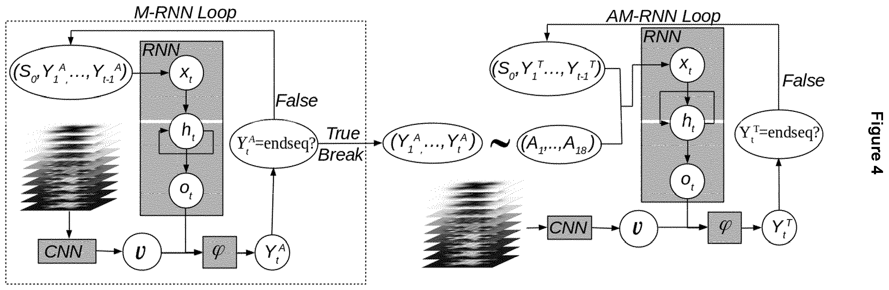

This attribute prediction starts with only the *startseq* token S₀ in the input (S₀, 0,..,0) for RNN/M-RNN_{A}. For a given time step t, we feed the RNN/M-RNN_{A} with the input (S₀, Y₁^{A} ,... ,Y^{A}ₜ₋₁, 0... ,0), that concatenates S0 with all the predictions already performed in previous time steps, and is padded with zeros until we obtain a length of 19 (see **Figure 3** for an example). The process is repeated until the *endseq* token is predicted and the loop is broken (see **Figure 4****).**

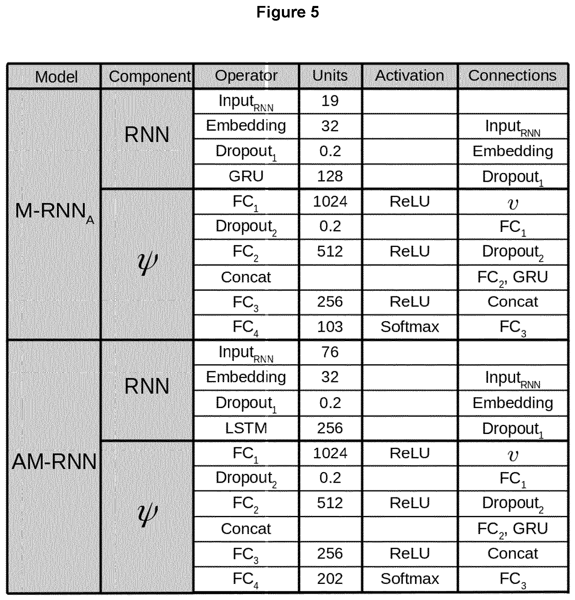

**Figure 5** provides details of the layers of the RNN/M-RNN_{A} and φ/M-RNN_{A} components of M-RNN_{A}, including the operator, dimensions and activation functions.

The global training of a complex network like M-RNN_{A} is extremely time consuming and prone to generate overfitting in the components with fewer layers. Therefore, the first trained Multimodal Recurrent Neural Network M-RNN_{A}, is trained in different stages:
Firstly, the first convolutional neural network CNN/M-RNN_{A} component feeding said first recurrent neural network CNN/M-RNN_{A} component is pretrained with a set of AFM images of known or predetermined molecules corresponding to a class of molecules that shares the same chemical composition, for instance with the AFM images of known or predetermined molecules corresponding to a class of molecules that shares the same chemical composition in the database QUAM-AFM, wherein the shape and the contrast of said images and their variation with the height show the 3D positions and the size (chemical nature) of the atoms and the distance between said atoms in the organic molecule.

Secondly, the first trained Multimodal Recurrent Neural Network M-RNN_{A} which first convolutional neural network CNN/M-RNN_{A} component is pre-trained with a plurality of constant-height AFM greyscale images of known or predetermined organic molecules, for instance with the AFM images of the database QUAM-AFM is fed and, alternatively,
- the RNN/M-RNN_{A} component and the CNN/M-RNN_{A} component are weighted fixing the weights of CNN/M-RNN_{A} component while training the RNN/M-RNN_{A} component,
- or the CNN/M-RNN_{A} component and the RNN/M-RNN_{A} component are weighted fixing the weight of RNN/M-RNN_{A} component while training the CNN/M-RNN_{A} component, to predict the IUPAC attributes in an organic molecule.

During training of the first trained Multimodal Recurrent Neural Network M-RNN_{A}, one of the 24 combinations of AFM operational parameters (6 different oscillation amplitudes, and 4 different values for the torsional stiffness of the CO molecule) available in QUAM-AFM for each input stack are randomly chosen. This variability in the input data
- makes sure that the parameters with which the AFM experiment has been carried out do not play a decisive role for the the first trained Multimodal Recurrent Neural Network M-RNN_{A} to succeed in the identification of the structure,
- prevents the overfitting, and
- provides the the first trained Multimodal Recurrent Neural Network M-RNN_{A} with the ability to generalize.

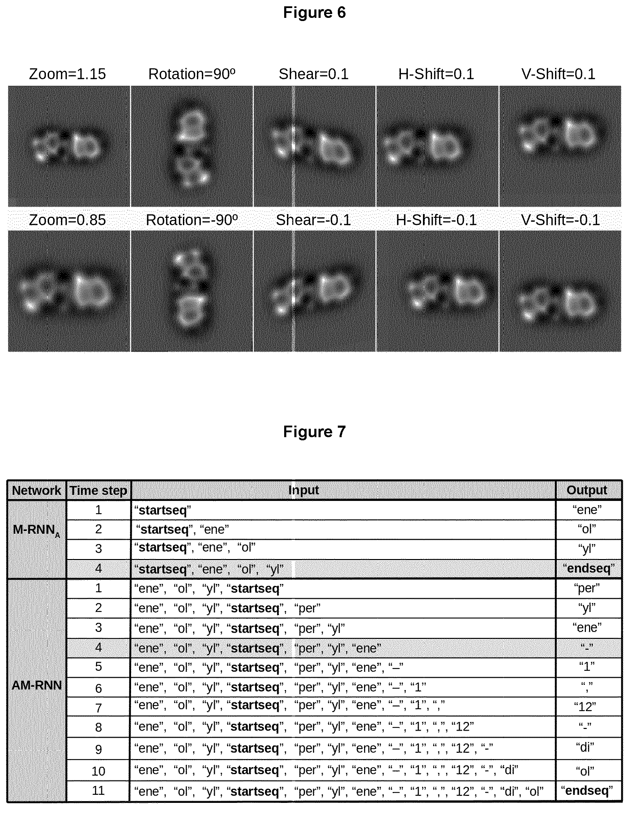

This variability is further enhanced with the application of an Imaging Data Generator (IDG) to the training set which applies different deformations (zoom, rotations, shifts, flips and shear) to the input images **(****Figure 6****)** and normalizes the pixel value. The use of an IDG is motivated by the fact that experimental images have some characteristic features that are not captured by AFM simulations and that could hamper identification. For example, experimental images do not display the full symmetry of the organic molecule. These differences between experimental and theoretical AFM images for a given organic molecule could be due to the unavoidable presence of noise in the experiments, asymmetries in the tip that are not included in the simulation of AFM images, or to the fact that organic molecules relax and deform due to the interaction with the substrate, while we are considering ideal, gas-phase structures in the simulated AFM images used for the training.

The deformations provided by the application of the IDG during the training mimic these effects and contribute significantly to confer the the first trained Multimodal Recurrent Neural Network M-RNN_{A} with the ability to identify organic molecules from experimental images. The selection of appropriate deformation parameters for the IDG is important as a proper choice considerably increases the accuracy of the identification.

The first trained Multimodal Recurrent Neural Network M-RNN_{A} comprises (see **Figure 2****)**
- a convolutional neural network CNN/M-RNN_{A} component comprises a block of 3D convolutional layers and one or more dropout layers configured to encode the plurality of constant-height Atomic Force Microscopy images into an output vector v,
- a recurrent neural network RNN/M-RNN_{A} component comprising one or more embedding layers, one or more dropout layers, and one or more recurrent layers, wherein at least one recurrent layer is a Gated Recurrent Unit (GRU) that deals with the language process, and is configured to embed a representation of each attribute based on its semantic meaning placing similar attributes close together and, secondly, to store the semantic temporal context in the recurrent layers,
- a multimodal component φ/M-RNN_{A} comprising connecting layers with one or more dropout layers, configured to combining and processing both CNN/M-RNN_{A} and RNN/M-RNN_{A} outputs to predict one of the attributes,
- and an Image Data Generator configured to apply pixel-value normalization and different random deformations selected from [-15,15] % for zoom, [-180,180] degrees of rotation, vertical and horizontal shift, random vertical and horizontal flips, [-20,20] % for shear and any combination thereof to the theoretical AFM images thereby providing mimic features of the experimental images like the presence of noise and effects associated to the tip asymmetry, that are absent in the AFM simulations and that could hamper identification.

A second trained Multimodal Recurrent Neural Network AM-RNN is used in the method of the present invention to obtain the name of the molecule according to the IUPAC nomenclature, predicting in the right order the different terms in the IUPAC name of the molecule using QUAM-AFM.

A method for training the second trained Multimodal Recurrent Neural Network AM-RNN comprising the following steps:
1) providing a second trained Multimodal Recurrent Neural Network AM-RNN, said AM-RNN comprising
   - a second convolutional neural network CNN/AM-RNN component comprising a block of 3D convolutional layers and one or more dropout layers,
   - a second recurrent neural network RNN/AM-RNN component comprising one or more embedding layers, one or more dropout layers, and one or more recurrent layers, wherein at least one recurrent layer is a Long-Short-Term Memory (LSTM),
   - a second multimodal component φ/M-RNN_{A} comprising connecting layers with one or more dropout layers,
   - and a second Image Data Generator
2) training the second convolutional neural network CNN/M-RNN_{A} component feeding said first convolutional neural network CNN/M-RNN_{A} component with a set of AFM images of known or predetermined molecules corresponding to a class of molecules that shares the same chemical composition, for instance with the AFM images of known or predetermined molecules corresponding to a class of molecules that shares the same chemical composition in the database QUAM-AFM, wherein the shape and the contrast of said images and their variation with the height show the 3D positions and the size (chemical nature) of the atoms and the distance between said atoms in the organic molecule
3) feeding the second trained Multimodal Recurrent Neural Network AM-RNN which first convolutional neural network CNN/AM-RNN component is trained in step (2) with a plurality of constant-height AFM greyscale images of known or predetermined organic molecules, for instance with the AFM images of the database QUAM-AFM and the IUPAC attributes obtained in the training of the first trained Multimodal Recurrent Neural Network M-RNN_{A}, and, alternatively,
   - weighting the RNN/AM-RNN component and the CNN/AM-RNN component fixing the weight of CNN/AM-RNN component while training the RNN/AM-RNN component,
   - or weighting the CNN/AM-RNN component and the RNN/AM-RNN component fixing the weight of RNN/AM-RNN component while training the CNN/AM-RNN component,
thereby generating the IUPAC name of the organic molecule.

The CNN/AM-RNN component comprises a block of 3D convolutional layers and dropout layers and consists of a modification of the Inception ResNet V2 model (C. Szegedy, S. loffe, V. Vanhoucke, A. A. Alemi, 31rd Proc. AAAI Conf. on Artificial Intelligence (AAAI Press, Palo Alto, CA, USA, 2017), p. 4278-4284) where the 2D-convolutional layers is replaced by a block that includes two 3D convolutional layers - each one with 32 filters, (3,3,3) kernel size and (2,1,1) strides- in order to process the stack of 10 AFM images with various tip--sample distances, followed by a dropout layer. This dropout layer is essential to generalize to different images, such as the experimental ones. In addition, we have removed the last fully connected layer of the model, which is specific for the original classification task, obtaining an output vector v.

The RNN/AM-RNN component includes an embedding layer, followed by a dropout layer, and ending with a recurrent layer (see **Figure 2****).** The terms are encoded by assigning integer numbers (from 1 to 199) to each term. The input of RNN/AM-RNN is a vector of fixed size 76. This number comes from the sum of the maximum number of different attributes in the names of the molecules in QUAM-AFM plus the *startseq* token (18+1=19), and the maximum number of terms in the IUPAC names of the molecules in QUAM-AFM is 57. Each RNN/AM-RNN input is a vector of size 76, arising from the concatenation of the attributes predicted by the first trained Multimodal Recurrent Neural Network M-RNN_{A} (padded with zeros if less than 18) with the *startseq* token and the terms predicted at each previous time step (padded until we obtain a vector with length 57). In the first step, it will contain the integer numbers designating the *startseq* token and attributes, while, in the following steps, it would also include the integer numbers associated with terms predicted in previous steps.

The embedding layer processes the input to represent each term in a vector space, transforming the input vector into a dense vector with real values that reflect the syntactic and semantic meaning of the term, placing similar inputs close together in the vector space, following the neural connections established during training. For example, the terms designating numbers are represented in close proximity, i.e. the terms closest to *nona* are *octa, deca, undeca* and *dodeca.*

The recurrent layer stores in its internal state information about the previous predictions. In term prediction, we use a Long Short-Term Memory (LSTM) network as the recurrent layer. LSTM, more accurate with long time series than the GRU used in RNN/M-RNN_{A}, is appropriate for the prediction of the longer terms strings, with a maximum length of 57 terms. Finally, it is necessary to introduce a dropout layer in between the embedding and recurrent layers in order to avoid overfitting during the training.

The multimodal component φ/AM-RNN first processes the CNN/M-RNN_{A} output v in two fully connected layers with a dropout between them. The resulting vector is concatenated with the output of the RNN/AM-RNN to feed two fully connected layers that produce a vector of probabilities. This vector has 202 components: 199 components are related to the terms, one with the padding and two with the *startseq* and *endseq* tokens. The position of the larger component in the vector provides us with the prediction of a new term.

The term prediction starts with the list of attributes predicted in step (b) (padded if necessary) plus the *startseq* token (Y₁^{A},...,Y^{A}₁₈, *startseq,*0*,...,*0) as the input for RNN/AM-RNN. For a given time step t, the RNN/AM-RNN is fed with the input (Y₁^{A} ,... ,Y^{A}₁₈, *startseq,*Y₁^{T} ,... ,Y^{T}ₜ₋₁, 0...,0), that includes all the term predictions already performed in previous time steps, padding with zeros to obtain a length of 57, the maximum number of terms. The process is repeated until the *endseq* token is predicted and the loop is broken (see **Figure 4****).** The inputs and outputs at each time step in the prediction of terms with AM-RNN for the perylene-1,12-diol molecule is shown in **Figure 7****.** The representation of the state of the RNN/AM-RNN and the input vector of AM-RNN in the fourth time step are displayed in **Figure 3****.**

**Figure 5** provides details of the layers of the RNN/AM-RNN and φ/AM-RNN components of AM-RNN, including the operator, dimensions and activation functions.

Firstly, the second convolutional neural network CNN/AM-RNN component feeding said second recurrent neural network CNN/AM-RNN component is pretrained with a set of AFM images of known or predetermined molecules corresponding to a class of molecules that shares same chemical composition, for instance with the AFM images of known or predetermined molecules corresponding to a class of molecules that shares the same chemical composition in the database QUAM-AFM, wherein the shape and the contrast of said images and their variation with the height show the 3D positions and the size (chemical nature) of the atoms and the distance between said atoms in the organic molecule.

Secondly, the second trained Multimodal Recurrent Neural Network AM-RNN which second convolutional neural network CNN/AM-RNN component is pre-trained with a plurality of constant-height AFM greyscale images of known or predetermined organic molecules, for instance with the AFM images of the database QUAM-AFM is fed and alternatively,
- the RNN/AM-RNN component and the CNN/AM-RNN component are weighted fixing the weight of CNN/AM-RNN component while training the RNN/AM-RNN component,
- or the CNN/AM-RNN component and the RNN/AM-RNN component are weight fixing the weight of RNN/AM-RNN component while training the CNN/AM-RNN component, thereby generating the IUPAC name of the organic molecule.

The second trained Multimodal Recurrent Neural Network AM-RNN comprises (see **Figure 2****)**
- a second convolutional neural network CNN/AM-RNN comprising a block of 3D convolutional layers and one or more dropout layers configured component configured to encode the plurality of constant-height Atomic Force Microscopy images obtained in step (a) into an output vector *v*;
- a second recurrent neural network RNN/AM-RNN component comprising one or more embedding layers, one or more dropout layers, and one or more recurrent layers, wherein at least one recurrent layer is a Long-Short-Term Memory (LSTM), configured to deal with the language process, and to embed a representation of each IUPAC term based on its semantic meaning placing similar terms close together and, secondly, to store the semantic temporal context in the recurrent layers;
- a second multimodal component φ/AM-RNN comprising connecting layers with one or more dropout layers, that is in charge of combining and processing both CNN/AM-RNN and RNN/AM-RNN outputs to predict one of the IUPAC terms;
- and a second Image Data Generator configured to apply pixel-value normalization and different random deformations selected from [-15,15] % for zoom, [-180,180] degrees of rotation, vertical and horizontal shift, random vertical and horizontal flips, [-20,20] % for shear and any combination thereof to the theoretical AFM images thereby providing mimic features of the experimental images like the presence of noise and effects associated to the tip asymmetry, that are absent in the AFM simulations and that could hamper identification.

Claim 1 of the present invention refers to a computer implemented method for identifying an organic molecule from Atomic Force Microscopy images and for generating the name of the organic molecule according to the IUPAC nomenclature, said method comprising the following steps:
a) acquiring a plurality of constant-height Atomic Force Microscopy images of an organic molecule at different tip height distances above said molecule using a functionalized metal tip apex by means of a Frequency Mode Atomic Force microscope (FM-AFM microscope), wherein said different tip height distances range between 280 pm and 370 pm above said molecule and wherein the shape and the contrast of said image and their variation with the tip height show the 3D position and, the size (chemical nature) of the atoms and the distance between said atoms in the organic molecule,
b) providing a first trained Multimodal Recurrent Neural Networks M-RNN_{A} to a data processor device, wherein the first trained Multimodal Recurrent Neural Networks M-RNN_{A} comprises
   - a first convolutional neural network CNN/RNN_{A} component comprising a block of 3D convolutional layers and one or more dropout layers
   - a first recurrent neural network component RNN/M-RNN_{A} component comprising one or more embedding layers, one or more dropout layers, and one or more recurrent layers, wherein at least one recurrent layer is a Gated Recurrent Unit (GRU), and
   - a first multimodal φ/AM-RNN component comprising connecting layers with one or more dropout layers,
c) feeding, to the data processor device, the first trained Multimodal Recurrent Neural Networks M-RNN_{A} with the atomic Force Microscopy images obtained in step (a), said first trained Multimodal Recurrent Neural Networks M-RNN_{A} generating the IUPAC attributes with syntactic and semantic meaning of the organic molecule;
d) providing a second trained Attribute Multimodal Recurrent Neural Network AM-RNN to the data processor device, wherein the second trained Attribute Multimodal Recurrent Neural Network AM-RNN comprises
   - a second convolutional neural network CNN/AM-RNN component comprising a block of 3D convolutional layers and one or more dropout layers,
   - a second recurrent neural network component RNN/ AM-RNN component comprising one or more embedding layers, one or more dropout layers, and one or more recurrent layers, wherein at least one recurrent layer is a Long-Short-Term Memory (LSTM), and
   - a second multimodal φ/AM-RNN component comprising connecting layers with one or more dropout layers,
e) feeding, to the data processing device, the second trained Multimodal Recurrent Neural Network AM-RNN with the IUPAC attributes obtained in step (d) and the atomic Force Microscopy images obtained in step (a), said second trained Multimodal Recurrent Neural Network AM-RNN generating the name of the IUPAC of the organic molecule.

AFM operating in its Frequency Modulation dynamic mode (FM-AFM) allows characterization and manipulation of all kinds of materials at the atomic scale by measuring the change in the frequency of an oscillating tip due to its interaction with the organic molecule sample. When the tip is functionalized with inert closed-shell molecules, in particular a CO molecule, the resolution is enhanced providing access to the inner structure of molecules. The outstanding contrast originates mainly from the Pauli repulsion between the CO probe and the sample molecule. This repulsive force contribution arises because the electron densities of tip and sample overlap, resulting in increasing frequency shifts, which are changes in the oscillation frequency of the cantilever holding the tip due to the tip-sample interaction. Shifts are observed as bright features in the constant height AFM images above atom positions, sizes and bonds (distance between the atoms), reflecting the organic molecular structure.

The term "a functionalized metal tip apex" refers herein to a tip apex of a metal, usually Cu but other metals like Ag and Pt can also be used, which is functionalized with inert closed shell atoms or molecules that dramatically enhanced the resolution of the AFM images, providing access to the inner structure of the organic molecule. Examples of inert closed shell atoms or molecules are a Xe atom and a CO molecule, respectively.

A CO functionalized metal tip apex is preferred in the present invention since it increases the AFM image contrast because of:
- the Pauli repulsion between the lone pair of the oxygen atom in the CO molecule and the charge density of the sample molecule is very directional, due to the preferential distribution of electronic charge associated with the lone pair along the molecular axis, and
- the CO molecule attached to the metal tip provides a complex electric field, with a very localized central feature, right in front of the O atom and repulsive for the electrons in the sample molecule, that provides a rapidly varying electrostatic interaction, both vertical and laterally, and
- the tilting of the CO molecule significantly enhances the intramolecular features.

Therefore, in a preferred embodiment of the present invention, the functionalized metal tip apex used in step (a) is selected from Cu, Ag or Pt.

In another preferred embodiment of the method of the present invention, the functionalized metal tip apex used in step (a) is functionalized with inert closed shell atoms or molecules. More preferably, the functionalized metal tip apex is functionalized with a Xe atom or a CO molecule.

For acquiring the plurality of constant-height AFM greyscale images different tip height distances above said organic molecule ranging between 280 pm and 370 pm have to be obtained by means of an FM-AFM microscope that allows the acquisition of images with different contrasts as a function of the tip height distances. The shape and the contrast of said images and their variation with the tip height defines the 3D spatial distribution of the electronic charge, that results from the interplay of the chemical species, their chemical environment, and their relative heights with respect to the other atoms in the molecular configuration Therefore, it is essential that a plurality of images is acquired since the whole information regarding said 3D spatial distribution of the electronic charge is therein. Preferably, at least 10 images should be taken in order to properly characterize the FM-AFM contrast in the tip height distance range between 280 pm and 370 pm above said organic molecule. In this tip height range, the Pauli repulsion and the electrostatic interaction between the tip and the organic molecule, vary significantly, leading to strong changes in the contrast of the FM-AFM images that include features that are characteristic of the atoms and their molecular environment. The shape and the contrast of said images and their variation with the tip height contains all the information about the 3D position and the size (chemical nature) of the atoms and the distance between said atoms.

Therefore, in a preferred embodiment of the method of the present invention, a plurality of at least 10 constant-height AFM greyscale images of the organic molecule are acquired in step (a).

In another preferred embodiment of the method of the present invention, step (a) is performed at least 10 different tip height distances.

Step (b) of the method of the invention refers to providing a first trained Multimodal Recurrent Neural Networks M-RNN_{A} to a data processor device, wherein the first trained Multimodal Recurrent Neural Networks M-RNN_{A} comprises
- a first convolutional neural network CNN/RNN_{A} component comprising a block of 3D convolutional layers and one or more dropout layers,
- a first recurrent neural network component *RNN*/*M-RNN_{A}* component comprising one or more embedding layers, one or more dropout layers, and one or more recurrent layers, wherein at least one recurrent layer is a Gated Recurrent Unit (GRU), and
- a first multimodal φ/*AM-RNN* component comprising connecting layers with one or more dropout layers,
and step c) feeding, to the data processor device, the first trained Multimodal Recurrent Neural Networks M-RNN_{A} with the atomic Force Microscopy images obtained in step (a), said first trained Multimodal Recurrent Neural Networks M-RNN_{A} generating the IUPAC attributes with syntactic and semantic meaning of the organic molecule. As mentioned before, the attributes are mentioned in Table 1.

In step (d) a second trained Attribute Multimodal Recurrent Neural Network AM-RNN is provided to the data processor device, wherein the second trained Attribute Multimodal Recurrent Neural Network AM-RNN comprises
- a second convolutional neural network CNN/AM-RNN component comprising a block of 3D convolutional layers and one or more dropout layers,
- a second recurrent neural network component RNN/ AM-RNN component comprising one or more embedding layers, one or more dropout layers, and one or more recurrent layers, wherein at least one recurrent layer is a Long-Short-Term Memory (LSTM), and
- a second multimodal φ/AM-RNN component comprising connecting layers with one or more dropout layers.

Step (e) refers to the feeding, to the data processing device, the second trained Multimodal Recurrent Neural Network AM-RNN with the IUPAC attributes obtained in step (d) and the atomic Force Microscopy images obtained in step (a), said second trained Miltimodal Recurrent Neural Network AM-RNN generating the name of the IUPAC of the organic molecule.

Claim 7 of the present invention refers to a Frequency Modulation Atomic Force Microscopy (FM-AFM) microscope comprising a functionalized metal tip apex (herein the microscope of the present invention) configured to carry out step (a) of the method of the present invention as described above and a data processing device configured to carry out steps (b) to step (e) of the method of the present invention as described above.

Preferably, the microscope of the present invention further comprising a display unit connected to the data processing device and configured to display the name of the molecule according to IUPAC obtained in step (e) of the method of the present invention. More preferably, the display unit connected to the data processing device is further configured to display the structural representation of the molecule identified in step (e) of the method of the present invention in the form of a ball and stick depiction.

In another preferred embodiment of the microscope of the present invention, the metal of the functionalized metal tip apex is selected from Cu, Ag or Pt. Said metal tip apex enhanced the resolution of the FM-AFM images.

In another preferred embodiment of the microscope of the present invention, the functionalized metal tip apex is functionalized with inert closed shell atoms or molecules, preferably the functionalized metal tip apex is functionalized with an inert closed shell Xe atom or an inert closed shell CO molecule. Said functionalized metal tip apex dramatically enhanced the resolution of the FM-AFM images.

Claim 13 of the present invention refers to a computer program (herein the computer program of the invention) comprising instructions which, when the program is executed by the data processing device comprised in the FM-AFM of the present invention as described above, cause said data processing device to carry out step (b) to step (e) of the method of the present invention as described above in said data processing device.

Claim 14 of the present invention refers to a computer-readable data carrier having stored thereon the computer program of the present invention as described above.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples and drawings are provided by way of illustration and are not intended to be limiting of the present invention.

### BRIEF DESCRIPTION OF THE DRAWINGS

**Figure 1** Atomic structures belonging to different classes according to their chemical species.
**Figure 2** shows the graphical layer representation of the common structure shared by both M-RNN_{A} and AM-RNN with the layers that constitute their three components, CNN, RNN and φ. The CNN component follows the Inception ResNet V2 model, where the first 2D-convolutional layers have been replaced by two 3D convolutional layers (to process the image stack), followed by a dropout layer (gray). The following blocks are just a pictorial representation of the complex architecture (with 164 layers) of the original Inception ResNet V2 model. Notice that the last fully connected layer of this model, which is specific for the original classification task, has been removed, obtaining an output vector (v) that would further process by the φ component. The RNN component includes embedding (white), dropout (gray), and recurrent (black) layers. The black box represents a GRU layer in M-RNN_{A}, whereas in the AM-RNN it represents a LSTM layer. The φ component process the output vector of the CNN component with two fully connected layers (white) with a dropout layer (gray) between them, concatenates this result with the output of the RNN component, and process it through another two fully connected layers that produce a vector of probabilities. The position of the larger component in the vector provides us with the prediction of a new attribute (term) in the case of M-RNN_{A} (AM-RNN). Figure 5 provides further details of the layers of the RNN and φ components of M-RNN_{A} and AM-RNN, including the operator, dimensions and activation functions.
**Figure 3** shows a representation of the RNN in the same format used in Figure 4, corresponding to the fourth time step in M-RNN_{A} and AM-RNN for the perylene-1,12-diol molecule. This figure highlights the fact that the state of the RNN, in particular, the recurrent layer, depends on the previous predictions.
**Figure 4** shows the architecture scheme of the method of the present invention com prising the combination of two different Multimodal Recurrent Neural Networks whose data flow are illustrated in: (a) M-RNN_{A} and (b) AM-RNN. Rectangular boxes represent the three components of each M-RNN: a convolutional neural network CNN, a recurrent neural network RNN, and the multimodal component φ. In the RNN component, xₜ represents the input at time step t, hₜ represents the internal state of the recurrent layer at t and oₜ is the output of RNN at t. The arrows indicate the flow of information within the model. M-RNN_{A} predicts an attribute at each time step until the loop is broken with the *endseq* token, whereas AM-RNN predicts the sorted terms (one at each time step) that give rise to the IUPAC name. Examples showing the inputs and the outputs at each time step predicted by the M-RNN_{A} and AM-RNN networks from a 3D image stack (taken from the QUAM-AFM data set) corresponding to the perylene-1,12-diol molecule are shown in Figure 7. A representation of the RNN corresponding to the fourth time step in M-RNN_{A} and AM-RNN for the perylene-1,12-diol molecule can be found in Figure 3.
**Figure 5** shows the layer-by-layer details of the RNN and φ components integrated in M-RNN_{A} and AM-RNN. v denotes the output vector of the CNN component. The operators are the same for both models, except for the last layer of the RNN, a GRU in M-RNNA (attribute prediction) and an LSTM in AM-RNN (term prediction). However, the units are different as corresponds to the different size of the inputs and outputs in each model.
**Figure 6** shows the results of applying the Image Data Generator (IDG) to the same AFM image of a N-(5-amino-4-methylpyridin-2-yl)-6-fluoro-1-benzothiophene-2-carboxamide molecule. The parameters selected for the IDG during the training are randomly chosen in the range of [-180,180] degrees for rotation, [-15,15] % for zoom and for vertical and horizontal shift, [-20,20] % for shear and random vertical and horizontal flip. When necessary, nearest filling has been applied for the points outside of the boundaries of the input. A proper selection of the range of parameters for the IDG used during training considerably increases the accuracy of the IUPAC name predicted applying the method of the invention to experimental images.
**Figure 7** shows the inputs and the outputs at each time step predicted by the M-RNN_{A} and AM-RNN networks from a 3D image stack (taken from the QUAM-AFM data set) corresponding to the case of a perylene-1,12- diol molecule. The t = 4 time steps of both networks, analyzed in detail in Figure 3 are highlighted in gray.

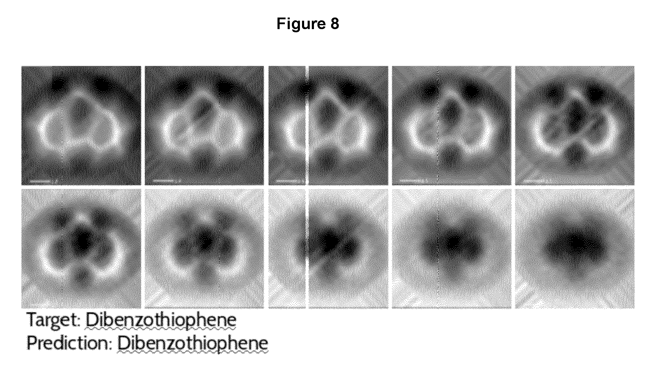

**Figure 8** shows AFM experimental images of dibenzothiophene taken at different tip-sample distances (from P. Zahl, Y. Zhang, Energy Fuels 33, 4775 (2019). Despite the strong noise and the white lines crossing the images diagonally, the method of the present invention provides a perfect prediction of the IUPAC name.

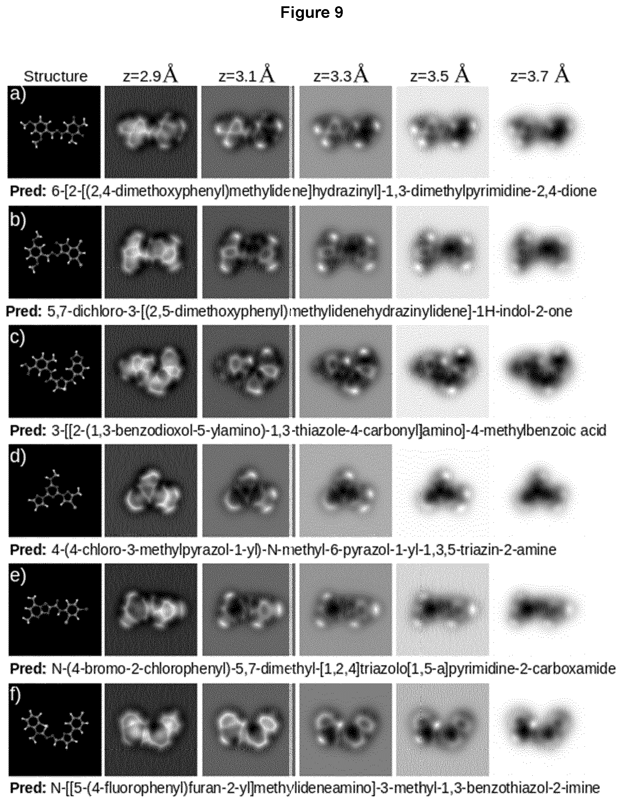

**Figure 9** shows examples of perfect predictions for molecules containing carbon, nitrogen, oxygen and different halogen atoms. Each subfigure shows (from left to right), a ball- and-stick depiction (generated with the open-source code Jmol) of the molecular structure and five AFM images at various tip-sample distances. The IUPAC name predicted by the the method of the present invention, that matches exactly the ground truth, is shown below the images.

### EXAMPLES

### EXAMPLE 1: Prediction of the IUPAC name from experimental AFM images for dibenzothiophene

The example is based on experimental AFM images for dibenzothiophene adsorbed on Au (111) measured using a CO-functionalized tip in the constant height mode. The measurements were performed with a Createc-based low-temperature (LT)-STM upgraded with AFM and using a custom GXSM control software. A Q-Plus sensor (operated at 30 kHz with a typical Q factor around 10 000) with a focused ion beam (FIB)-sharpened Ptlr tip wire functionalized with a CO molecule was used. The AFM was operated in a constant height mode, with feedback off with no corrections needed within about 1 h and more. XYZ drift and creep was less than 1 pm/h after at least 24 h at 5 K and without major XYZ offset changes. Molecules were imaged with bimodal STM and AFM under UHV conditions at 5 K after deposition using a home-built evaporator.

Regarding operation conditions, the tip oscillation amplitude was approximately 50 pm and a bias voltage of 40 mV was applied. This bias minimizes the electrostatic force between the Au(111) surface and the actual tip apex and provides stable imaging conditions. For frequency tracking and amplitude regulation, a novel phase amplitude convergence detector and phase-locked loop were used. The Au(111) single crystal was used here as the substrate and cleaned with typically 3 cycles of Ar+ sputter/anneal before use. Tip tuning was performed via controlled crashing into the Au surface, following CO molecule functionalization, via close proximity scanning and pickup at very low bias.

**Figure 8** shows a set of 10 AFM images taken at different tip-sample distances for dibenzothiophene adsorbed on Au (111)

The M-RNN_{A}, trained with the QUAM-AFM data set as described above, was applied to the stack of 10 constant-height AFM images shown in **Figure 8****.** M-RNN_{A} correctly produced the attributes: *benz, phen* and *thi.*

The AM-RNN, trained with the QUAM-AFM data set as described above, was feed with the stack of 10 constant-height AFM images shown in Figure 1 and the attributes (*benz, phen* and *thi.*) determined by M-RNN_{A}. AM-RNN predicted the terms in the molecule's name in the correct order: *di, benz, o, thi, o, phen* and *e*. It has to be noticed that, despite the strong noise and the white lines crossing the images diagonally, we obtain a perfect prediction.

### EXAMPLE 2: Prediction of the IUPAC name from theoretically simulated AFM images taken from the QUAM-AFM data set

This second example demonstrates the ability of our method to predict the IUPAC name of non-flat molecules with a complex structure and composition that include most of the relevant chemical species in organic chemistry. In this case, we feed the model of the invention with theoretically simulated AFM images taken from the QUAM-AFM data set. These images have been calculated with a torsional stiffness of 0.40 N/m, and an oscillation amplitude of 40 pm. None of the three molecules considered have been shown to either M-RNNA or AM-RNN during their training. For each of the molecule, we consider a stack of 10 constant-height AFM images calculated at different tip heights in the range from 280 pm to 370 pm with increments of 10 pm between the images.

**Figure 9** shows 5 of the stack of 10 constant-height AFM images for each of the molecules studied together with their ball-and-stick depiction, generated with Jmol, an open-source Java viewer for chemical structures (http://www.jmol.org/), in order to illustrate graphically their structure and composition.

For the case shown in **Figure 9a****,** the trained M-RNNA correctly produced the attributes for this molecule. The attributes are (in alphabetical order): *azin, hydr, ide, imidin, meth, one, oxy, phen, pyr* and *yl.* The trained AM-RNN predicted all the terms and in the correct order to form the IUPAC name in all of the cases. Notice that this means that the method of the present invention has identified from the images, without any error, all the molecular moieties and it has also provided the exact IUPAC name, character by character.

Considering the molecules in this example, the method of the present invention is able to identify planar hydrocarbons, both cyclic or aliphatic, but also more complex structures as those including nitrogen or oxygen atoms that, due to their fast charge density decay, usually appear on the images as faint features.

Halogens, characterized on the images by oval features whose size and intensity are proportional to their σ-hole strength, have been also correctly labeled **(****Figures 9b, 9d and 9e****).**

The method of the present invention is even able to recognize the presence of the fluorine element, that does not induce a σ-hole and, when bonded to a carbon atom, produces an AFM fingerprint that is very similar to the one of a carbonyl group (compare **Figure 9e** with **Figure 9f****).** Hydrogen positions are often guessed, what is striking since hydrogen atoms bonded to *sp*² carbon atoms are hardly detected by the high-resolution AFM (HR-AFM) due to their negligible charge density.

### EXAMPLE 3: COMPARATIVE EXAMPLE

A single M-RNN was fed with a stack of AFM images to predict the IUPAC name of the imaged molecule. We have followed this route, with a model completely analogous to a AM-RNN. The only difference is that the input of the RNN component is a vector of length 58, that in the first step includes only the the *startseq* token. The M-RNN was trained in three stages, fixing alternatively the weights of the CNN and RNN components. This single M-RNN provided 25 predicting the IUPAC names.

## Claims

1. A computer implemented method for identifying an organic molecule from Atomic Force Microscopy images and for generating the name of the organic molecule according to the IUPAC nomenclature, said method comprising the following steps:
a) acquiring a plurality of constant-height Atomic Force Microscopy images of an organic molecule at different tip height distances above said organic molecule using a functionalized metal tip apex by means of a Frequency Mode Atomic Force microscope , wherein said different tip height distances range between 280 pm and 370 pm and wherein the shape and the contrast of said image and their variation with the tip height show the 3D position of the atoms, the size of the atoms and the distance between said atoms in the organic molecule;
b) providing a first trained Multimodal Recurrent Neural Networks M-RNN_{A} to a data processor device, wherein the first trained Multimodal Recurrent Neural Networks M-RNN_{A} comprises
- a first convolutional neural network CNN/RNN_{A} component comprising a block of 3D convolutional layers and one or more dropout layers
- a first recurrent neural network component *RNN*/*M-RNN_{A}* component comprising one or more embedding layers, one or more dropout layers, and one or more recurrent layers, wherein at least one recurrent layer is a Gated Recurrent Unit (GRU), and
- a first multimodal φ/*AM-RNN* component comprising connecting layers with one or more dropout layers,
c) feeding, to the data processor device, the first trained Multimodal Recurrent Neural Networks M-RNN_{A} with the atomic Force Microscopy images obtained in step (a), said first trained Multimodal Recurrent Neural Networks M-RNN_{A} generating the IUPAC attributes with syntactic and semantic meaning of the organic molecule;
d) providing a second trained Attribute Multimodal Recurrent Neural Network AM-RNN to the data processor device, wherein the second trained Multimodal Recurrent Neural Network AM-RNN comprises
- a second convolutional neural network CNN/*AM-RNN* component comprising a block of 3D convolutional layers and one or more dropout layers,
- a second recurrent neural network component *RNN*/ *AM-RNN* component comprising one or more embedding layers, one or more dropout layers, and one or more recurrent layers, wherein at least one recurrent layer is a Long-Short-Term Memory (LSTM), and
- a second multimodal *φ*/*AM-RNN* component comprising connecting layers with one or more dropout layers,
e) feeding, to the data processing device, the second trained Multimodal Recurrent Neural Network AM-RNN with the IUPAC attributes obtained in step (d) and the Atomic Force Microscopy images obtained in step (a), said second trained Multimodal Recurrent Neural Network AM-RNN generating the name of the IUPAC of the organic molecule.

2. The method according to claim 1, wherein a plurality of at least 10 constant-height Atomic force Microscopy images of the organic molecule are acquired in step (a).

3. The method according to any of claims 1 or 2, wherein step (a) is performed at at least 3 different height distances, preferably at at least 10 different tip height distances.

4. The method according to any of claims 1 to 3, wherein the functionalized metal tip apex used in step (a) is selected from Cu, Ag or Pt.

5. The method according to any of claims 1 to 4, wherein the functionalized metal tip apex used in step (a) is functionalized with inert closed shell atoms or molecules.

6. The method according to any of claims 1 to 5, wherein the functionalized metal tip apex used in step (a) is functionalized with a Xe atom or a CO molecule.

7. A Frequency Modulation Atomic Force Microscopy (FM-AFM) microscope comprising a functionalized metal tip apex and configured to carry out the step (a) of the method according to any of claims 1 to 6 and a data processing device configured to carry out steps (b) to step (e) of the method according to any of claims 1 to 6.

8. The FM-AFM microscope according to claim 7, further comprising a display unit connected to the data processing device and configured to display the name of the molecule according to IUPAC obtained in step (e) of the method according to any of claims 1 to 6.

9. The FM-AFM microscope according to any of claims 7 or 8, wherein the display unit connected to the data processing device is further configured to display the structural representation of the molecule identified in step (e) of the method according to any of claims 1 to 6 in the form of a ball and stick depiction.

10. The FM-AFM microscope according to any of claims 7 to 9, wherein the metal of the functionalized metal tip apex is selected from Cu, Ag or Pt.

11. The FM-AFM microscope according to any of claims 7 to 10, wherein the functionalized metal tip apex is functionalized with inert closed shell atoms or molecules.

12. The FM-AFM microscope according to any of claims 7 to 11, wherein the functionalized metal tip apex is functionalized with a Xe atom or a CO molecule.

13. A computer program comprising instructions which, when the program is executed by the data processing device comprised in the FM-AFM of any of claims 7 to 12, cause said data processing device to carry out steps (b) to (e) according to the method of claims 1 to 6, in said data processing device.

14. A computer-readable data carrier having stored thereon the computer program of claim 13.

## Patentansprüche

1. Computerimplementiertes Verfahren zur Identifizierung eines organischen Moleküls aus Bildern der Rasterkraftmikroskopie und zur Erzeugung des Namens des organischen Moleküls gemäß der IUPAC-Nomenklatur, wobei das Verfahren die folgenden Schritte umfasst:
a) Erfassen einer Vielzahl von Bildern der Rasterkraftmikroskopie mit konstanter Höhe eines organischen Moleküls bei unterschiedlichen Spitzenhöhenabständen über dem organischen Molekül unter Verwendung eines funktionalisierten Metallspitzenapex mittels eines Frequenzmodus-Rasterkraftmikroskops, wobei die unterschiedlichen Spitzenhöhenabstände im Bereich zwischen 280 pm und 370 pm liegen und wobei die Form und der Kontrast des Bildes und deren Variation mit der Spitzenhöhe die 3D-Position der Atome, die Größe der Atome und den Abstand zwischen den Atomen im organischen Molekül zeigen;
b) Bereitstellen eines ersten trainierten multimodalen rekurrenten neuronalen Netzwerks M-RNN_{A} für eine Datenverarbeitungsvorrichtung, wobei das erste trainierte multimodale rekurrente neuronale Netzwerk M-RNN_{A} Folgendes umfasst:
- eine erste Komponente eines neuronalen Faltungsnetzwerks CNN/RNN_{A}, die einen Block aus 3D-Faltungsschichten und eine oder mehrere Dropout-Schichten umfasst
- eine erste Komponente eines rekurrenten neuronalen Netzwerks *RNN*/*M-RNN_{A},* die eine oder mehrere Einbettungsschichten, eine oder mehrere Dropout-Schichten und eine oder mehrere rekurrente Schichten umfasst, wobei mindestens eine rekurrente Schicht eine Gated Recurrent Unit (GRU) ist, und
- eine erste multimodale *φ*/*AM-RNN-*Komponente, die Verbindungsschichten mit einer oder mehreren Dropout-Schichten umfasst,
c) Zuführen der ersten trainierten multimodalen rekurrenten neuronalen Netzwerke M-RNN_{A} mit den in Schritt (a) erhaltenen Bildern der Rasterkraftmikroskopie an die Datenverarbeitungsvorrichtung, wobei die ersten trainierten multimodalen rekurrenten neuronalen Netzwerke M-RNN_{A} die IUPAC-Attribute mit syntaktischer und semantischer Bedeutung des organischen Moleküls erzeugen;
d) Bereitstellen eines zweiten trainierten multimodalen rekurrenten neuronalen Netzwerks für Attribute AM-RNN für die Datenverarbeitungsvorrichtung, wobei das zweite trainierte multimodale rekurrente neuronale Netzwerk AM-RNN Folgendes umfasst:
- eine zweite Komponente eines neuronalen Faltungsnetzwerkes CNN/*AM-RNN,* die einen Block aus 3D-Faltungsschichten und einer oder mehreren Dropout-Schichten umfasst,
- eine zweite Komponente eines rekurrenten neuronalen Netzwerks *RNN*/*AM-RNN,* die eine oder mehrere Einbettungsschichten, eine oder mehrere Dropout-Schichten und eine oder mehrere rekurrente Schichten umfasst, wobei mindestens eine rekurrente Schicht ein langes Kurzzeitgedächtnis (LSTM) ist, und
- eine zweite multimodale *φ*/*AM-RNN-*Komponente, die Verbindungsschichten mit einer oder mehreren Dropout-Schichten umfasst,
e) Zuführen des zweiten trainierten multimodalen rekurrenten neuronalen Netzwerks AM-RNN mit den in Schritt (d) erhaltenen IUPAC-Attributen und den in Schritt (a) erhaltenen Bildern der Rasterkraftmikroskopie an die Datenverarbeitungsvorrichtung, wobei das zweite trainierte multimodale rekurrente neuronale Netzwerk AM-RNN den Namen der IUPAC des organischen Moleküls erzeugt.

2. Verfahren nach Anspruch 1, wobei eine Vielzahl von mindestens 10 Bildern der Rasterkraftmikroskopie mit konstanter Höhe des organischen Moleküls in Schritt (a) erfasst werden.

3. Verfahren nach einem der Ansprüche 1 oder 2, wobei Schritt (a) bei mindestens 3 verschiedenen Höhenabständen, vorzugsweise bei mindestens 10 verschiedenen Spitzenhöhenabständen, durchgeführt wird.

4. Verfahren nach einem der Ansprüche 1 bis 3, wobei der in Schritt (a) verwendete funktionalisierte Metallspitzenapex aus Cu, Ag oder Pt ausgewählt ist.

5. Verfahren nach einem der Ansprüche 1 bis 4, wobei der in Schritt (a) verwendete funktionalisierte Metallspitzenapex mit inerten Atomen oder Molekülen mit geschlossener Schale funktionalisiert ist.

6. Verfahren nach einem der Ansprüche 1 bis 5, wobei der in Schritt (a) verwendete funktionalisierte Metallspitzenapex mit einem Xe-Atom oder einem CO-Molekül funktionalisiert ist.

7. Mikroskop der Frequenzmodulations-Rasterkraftmikroskopie (FM-AFM), umfassend einen funktionalisierten Metallspitzenapex und dazu konfiguriert, Schritt (a) des Verfahrens nach einem der Ansprüche 1 bis 6 durchzuführen, und eine Datenverarbeitungsvorrichtung, die dazu konfiguriert ist, die Schritte (b) bis Schritt (e) des Verfahrens nach einem der Ansprüche 1 bis 6 durchzuführen.

8. FM-AFM-Mikroskop nach Anspruch 7, ferner umfassend eine Anzeigeeinheit, die mit der Datenverarbeitungsvorrichtung verbunden und dazu konfiguriert ist, den Namen des Moleküls gemäß IUPAC anzuzeigen, der in Schritt (e) des Verfahrens nach einem der Ansprüche 1 bis 6 erhalten wird.

9. FM-AFM-Mikroskop nach einem der Ansprüche 7 oder 8, wobei die mit der Datenverarbeitungsvorrichtung verbundene Anzeigeeinheit ferner dazu konfiguriert ist, die strukturelle Darstellung des Moleküls, das in Schritt (e) des Verfahrens nach einem der Ansprüche 1 bis 6 identifiziert wird, in Form einer Kugel-Stab-Darstellung anzuzeigen.

10. FM-AFM-Mikroskop nach einem der Ansprüche 7 bis 9, wobei das Metall des funktionalisierten Metallspitzenapex aus Cu, Ag oder Pt ausgewählt ist.

11. FM-AFM-Mikroskop nach einem der Ansprüche 7 bis 10, wobei der funktionalisierte Metallspitzenapex mit inerten Atomen oder Molekülen mit geschlossener Schale funktionalisiert ist.

12. FM-AFM-Mikroskop nach einem der Ansprüche 7 bis 11, wobei der funktionalisierte Metallspitzenapex mit einem Xe-Atom oder einem CO-Molekül funktionalisiert ist.

13. Computerprogramm, umfassend Anweisungen, die, wenn das Programm von der in dem FM-AFM nach einem der Ansprüche 7 bis 12 enthaltenen Datenverarbeitungsvorrichtung ausgeführt wird, die Datenverarbeitungsvorrichtung veranlassen, die Schritte (b) bis (e) gemäß dem Verfahren nach den Ansprüchen 1 bis 6 in der Datenverarbeitungsvorrichtung durchzuführen.

14. Computerlesbarer Datenträger, auf dem das Computerprogramm nach Anspruch 13 gespeichert ist.

## Revendications

1. Procédé mis en œuvre par ordinateur pour l'identification d'une molécule organique à partir d'images de microscopie à force atomique et la génération du nom de la molécule organique selon la nomenclature IUPAC, ledit procédé comprenant les étapes suivantes :
a) acquérir une pluralité d'images de microscopie à force atomique à hauteur constante d'une molécule organique à différentes distances de hauteur de pointe au-dessus de ladite molécule organique à l'aide d'une apex de pointe métallique fonctionnalisée au moyen d'un microscope à force atomique en mode fréquence, dans lequel lesdites différentes distances de hauteur de pointe sont comprises entre 280 pm et 370 pm et dans lequel la forme et le contraste de ladite image et leur variation en fonction de la hauteur de pointe indiquent la position 3D des atomes, la taille des atomes et la distance entre lesdits atomes dans la molécule organique ;
b) fournir des premiers réseaux neuronaux récurrents multimodaux M-RNN_{A} entraînés à un dispositif de traitement de données, dans lequel les premiers réseaux neuronaux récurrents multimodaux M-RNN_{A} entraînés comprennent
- un premier composant de réseau neuronal convolutif CNN/RNN_{A} comprenant un bloc de couches convolutives 3D et une ou plusieurs couches d'abandon
- un premier composant de réseau neuronal récurrent *RNN*/*M-RNN_{A}* comprenant une ou plusieurs couches d'incorporation, une ou plusieurs couches d'abandon et une ou plusieurs couches récurrentes, dans lequel au moins une couche récurrente est une unité récurrente à portes (GRU), et
- un premier composant *φ*/*AM-RNN* multimodal comprenant des couches de connexion avec une ou plusieurs couches d'abandon,
c) fournir, au dispositif de traitement des données, les premiers réseaux neuronaux récurrents multimodaux M-RNN_{A} entraînés avec les images de microscopie à force atomique obtenues à l'étape (a), lesdits premiers réseaux neuronaux récurrents multimodaux M-RNN_{A} entraînés générant les attributs IUPAC avec la signification syntaxique et sémantique de la molécule organique ;
d) fournir un second réseau neuronal récurrent multimodal d'attributs (AM-RNN) entraîné au dispositif de traitement de données, dans lequel le second réseau neuronal récurrent multimodal AM-RNN entraîné comprend
- un second composant de réseau neuronal convolutif *CNN*/*AM-RNN* comprenant un bloc de couches convolutives 3D et une ou plusieurs couches d'abandon,
- un second composant de réseau neuronal récurrent *RNN*/*AM-RNN* comprenant une ou plusieurs couches d'incorporation, une ou plusieurs couches d'abandon et une ou plusieurs couches récurrentes, dans lequel au moins une couche récurrente est une mémoire à long et court terme (LSTM), et
- un second composant *φ*/*AM-RNN* multimodal comprenant des couches de connexion avec une ou plusieurs couches d'abandon,
e) fournir, au dispositif de traitement de données, le second réseau neuronal récurrent multimodal AM-RNN entraîné avec les attributs IUPAC obtenus à l'étape (d) et les images de microscopie à force atomique obtenues à l'étape (a), ledit second réseau neuronal récurrent multimodal AM-RNN entraîné génère le nom de l'IUPAC de la molécule organique.

2. Procédé selon la revendication 1, dans lequel une pluralité d'au moins 10 images de microscopie à force atomique à hauteur constante de la molécule organique sont acquises à l'étape (a).

3. Procédé selon l'une quelconque des revendications 1 ou 2, dans lequel l'étape (a) est réalisée à au moins 3 distances de hauteur différentes, de préférence à au moins 10 distances de hauteur de pointe différentes.

4. Procédé selon l'une quelconque des revendications 1 à 3, dans lequel l'apex de pointe métallique fonctionnalisée utilisé à l'étape (a) est choisi parmi Cu, Ag ou Pt.

5. Procédé selon l'une quelconque des revendications 1 à 4, l'apex de pointe métallique fonctionnalisée utilisé à l'étape (a) est fonctionnalisé avec des atomes ou des molécules inertes à coquille fermée.

6. Procédé selon l'une quelconque des revendications 1 à 5, dans lequel l'apex de pointe métallique fonctionnalisée utilisé à l'étape (a) est fonctionnalisé avec un atome de Xe ou une molécule de CO.

7. Microscope de microscopie à force atomique à modulation de fréquence (FM-AFM) comprenant un apex de pointe métallique fonctionnalisée et configuré pour réaliser l'étape (a) du procédé selon l'une quelconque des revendications 1 à 6 et un dispositif de traitement de données configuré pour réaliser les étapes (b) à l'étape (e) du procédé selon l'une quelconque des revendications 1 à 6.

8. Microscope FM-AFM selon la revendication 7, comprenant en outre une unité d'affichage connectée au dispositif de traitement de données et configurée pour afficher le nom de la molécule selon l'IUPAC obtenu à l'étape (e) du procédé selon l'une quelconque des revendications 1 à 6.

9. Microscope FM-AFM selon l'une quelconque des revendications 7 ou 8, dans lequel l'unité d'affichage connectée au dispositif de traitement de données est en outre configurée pour afficher la représentation structurelle de la molécule identifiée à l'étape (e) du procédé selon l'une quelconque des revendications 1 à 6 sous la forme d'une représentation en boules et bâtonnets.

10. Microscope FM-AFM selon l'une quelconque des revendications 7 à 9, dans lequel le métal de l'apex de pointe métallique fonctionnalisée est choisi parmi Cu, Ag ou Pt.

11. Microscope FM-AFM selon l'une quelconque des revendications 7 à 10, l'apex de pointe métallique fonctionnalisée est fonctionnalisé avec des atomes ou des molécules inertes à coquille fermée.

12. Microscope FM-AFM selon l'une quelconque des revendications 7 à 11, dans lequel l'apex de pointe métallique fonctionnalisée est fonctionnalisé avec un atome de Xe ou une molécule de CO.

13. Programme informatique comprenant des instructions qui, lorsque le programme est exécuté par le dispositif de traitement de données compris dans le FM-AFM selon l'une quelconque des revendications 7 à 12, amènent ledit dispositif de traitement de données à réaliser les étapes (b) à (e) selon le procédé des revendications 1 à 6, dans ledit dispositif de traitement de données.

14. Support de données lisible par ordinateur sur lequel est stocké le programme informatique selon la revendication 13.
